Europäisches Patentamt

European Patent Office

Office européen des brevets

(19)

(11) EP 0 844 477 A1

## (12) EUROPEAN PATENT APPLICATION

(43) Date of publication:
**27.05.1998 Bulletin 1998/22**

(51) Int Cl.$^6$: **G01N 27/12**

(21) Application number: **97309379.2**

(22) Date of filing: **20.11.1997**

(84) Designated Contracting States:
**AT BE CH DE DK ES FI FR GB GR IE IT LI LU MC NL PT SE**
Designated Extension States:
**AL LT LV MK RO SI**

(30) Priority: **22.11.1996 US 755399**

(71) Applicant: **PITTWAY CORPORATION**
**Chicago, Illinois 60606 (US)**

(72) Inventors:
• **Tice, Lee D.**
**Bartlett, Illinois 60103 (US)**
• **Keeler, Manley**
**Naperville, Illinois 60565 (US)**

(74) Representative: **O'Connell, David Christopher**
**Haseltine Lake & Co.,**
**Imperial House,**
**15-19 Kingsway**
**London WC2B 6UD (GB)**

### (54) Detector with variable resistance sensor

(57)     An ambient condition sensor exhibits a resistance change as a function of the sensed condition. This change varies over several orders of magnitude. The sensor can be coupled to a circuit with a dynamically changing impedance to facilitate an accurate measurement of the variable sensor resistance. This circuit can include a ladder network of resistors which can be controllably coupled to the sensor as a function of sensor output resistance. The output signal from the sensor can be processed using analog or digital techniques to minimize noise induced variations. The sensor output resistance value can be converted to time for purposes of measurement and subsequent processing.

FIG. 1

## Description

### Field of the Invention:

The invention pertains to ambient condition detectors. More particularly, the invention pertains to such detectors which incorporate ambient condition sensors that vary over a large dynamic range and wherein the outputs of the sensors are processed so as to determine the existence of a predetermined ambient condition.

### Background of the Invention:

Various kinds of sensors are known for use in detecting the presence of predetermined ambient conditions. Representative sensors include ionization-type smoke sensors, photoelectric smoke sensors, gas sensors based on electrochemical cells as well as various types of solid state gas sensors.

One known type of solid state sensor is described in U.S. Patent 4,381,922 entitled *Combustion Detecting Device Using Metallo-Phthalocynine Semiconductor and Process of Preparing Same.* Sensors of the type disclosed in the '922 patent exhibit a variable resistance characteristic in the presence of combustion products and/or airborne pollutants associated with combustion.

The resistance characteristics of such sensors are known, for example, to fall in the presence of combustion products from smoldering fires and to increase into the presence of combustion products from flaming fires. One known type of circuit use the sensors of the type disclosed in the '922 patent detects a rate of change or slope of the changing resistance value for purposes of determining the presence of a predetermined condition.

In some instances, pollutants or products of combustion accumulate at a relatively slow rate and may not result in a great enough rate of change of resistance to be detected readily. Additionally, variations in absolute resistance values of a given sensor may not be indicative of the presence of combustion products or effects resulting from combustion but rather might be indicative of varying background levels of pollution.

There is thus a continuing need for circuitry which can process the outputs of variable resistance detectors to take into account varying rates of change of resistance values along with change in relative magnitudes of resistance values in response to ambient conditions.

### Summary of the Invention:

An apparatus for detecting the presence of a predetermined ambient condition includes a sensor having a resistance characteristic which varies in accordance with the ambient condition. Coupled to the sensor is processing circuitry useable to analyze the sensor outputs.

Sensor outputs can be processed by carrying out a plurality of smoothing functions. In one aspect the smoothing functions can be implemented using exponential-type smoothing or filtering. The smoothing or filtering can be carried out using either hard-wired circuitry or circuitry which includes programmable control units.

The raw sensor output is initially processed by smoothing same so as to produce a first smoothed output. The first smoothed output can be processed so as to produce a second smoothed output. The second smoothed output can in turn be processed to produce a third smoothed output. In each case, the smoothing processes are carried out with increasing time constants such that the third smoothed output exhibits a longer response time than do either the first or the second smoothed outputs.

Further processing can be carried out on the smoothed outputs by forming ratios between the first and second smoothed outputs. This ratio is indicative of the presence of a change in slope of the sensor output. In another aspect of the invention, a second ratio between the first smoothed output and the third smoothed output can be formed. This second ratio is indicative of the presence of a relative magnitude change in resistance value of the sensor.

In yet another aspect, the first and second ratios can be multiplied together to produce a processed output. As described above, this output reflects both changes in slope and changes in magnitude of the sensor's resistance value. The processed output can in turn be compared to one or more threshold values to determine whether or not a pre-alarm or an alarm condition is present.

In yet another aspect, the sensor can be incorporated into a variable frequency oscillator. The oscillator in turn produces an output signal with a frequency indicative of a substantially instantaneous resistance value being exhibited by the sensor.

The sensor can also be incorporated into circuitry which will in turn produce an electrical signal having a pulse width indicative of resistance value hence providing a resistance to time conversion. In a further aspect, the sensor can be coupled to a fixed resistor so as to form a voltage divider or resister divider circuit which in turn produces a divided output voltage indicative of sensor resistance value.

In a further embodiment, the output signals indicative of sensor resistance value can in turn be processed as described above in either hard-wired circuitry or by a programmable processor or control unit.

Numerous other advantages and features of the present invention will become readily apparent from the following detailed description of the invention and the embodiments thereof, from the claims and from the accompanying drawings.

### Brief Description of the Drawings:

Figure 1 is a block diagram illustrating a system in accordance with the present invention;

Figure 2 is a graph illustrating raw sensor output signals as well as smooth signals in accordance with the present invention;

Figure 3 is a schematic diagram of a variable frequency oscillator in a system in accordance with the present invention;

Figure 4 is a schematic diagram of a microprocessor-based resistance to time conversion circuit in ia system in accordance with the present invention;

Figure 5A, 5B together are a schematic diagram of a resister divider circuit in accordance with the present invention;

Figure 6 is a graph illustrating performance of a system, such as the system of Figure 1;

Figure 7 is a graph illustrating performance of a system, such as the system of Figure 1 in accordance with the present invention; and

Figure 8 is a subroutine illustrative of programmed steps for the purpose of carrying out a method in accordance with the present invention.

### Detailed Description of the Preferred Embodiments:

While this invention is susceptible of embodiment in many different forms, there are shown in the drawing and will be described herein in detail specific embodiments thereof with the understanding that the present disclosure is to be considered as an exemplification of the principles of the invention and is not intended to limit the invention to the specific embodiments illustrated.

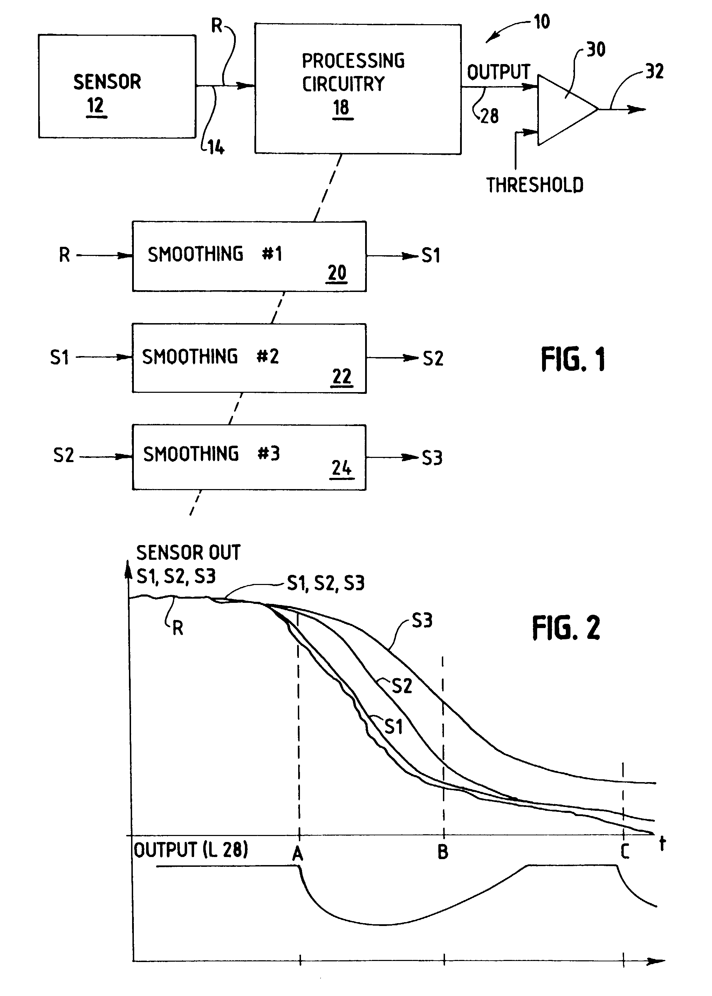

Figure 1 is a block diagram of a system 10 in accordance with the present invention. The system 10 includes a sensor 12 which could be a solid state sensor of a type taught by the above-noted U.S. Patent 4,381,922. Alternately, and without limitation, the sensor 12 could incorporate an electro-chemical sensor, a tinoxide sensor, an ionization-type or a photoelectric-type sensor without departing from the spirit and scope of the present invention. the output from the sensor 12 on a line 14 is an unprocessed electrical signal R which varies in response to a preselected, ambient characteristic such as gas, smoke, products of combustion or the like. The sensor 12 is in turn coupled to processing circuitry 18.

Processing circuitry 18 can be either hard wired or can include a programmable processor or control unit. Processor 18 carries out a set of functions so that a determination can be made as to whether a predetermined condition is present. Typical conditions include pre-alarm or alarm levels of pollution or products of combustion or the like.

Processing circuitry 18 carries out a plurality of smoothing steps indicated schematically at 20, 22, 24. It will be understood that the smoothing processes of elements 20-24 could be carried out in hard-wired circuitry, such as RC-type exponential filters or could be implemented in a programmable control unit, in digital form, such as by carrying out exponential-type smoothing or by forming long-term running averages.

In an initial smoothing function 20, the sensor output R is smoothed by the circuitry 18 so as to produce a smoothed output S1. Smoothed output S1 is in turn smoothed in element 22 thereby producing a second smoothed output S2. Finally, second smoothed output S2 is in turn smoothed one more time in element 34 thereby producing third smoothed output S3.

It will be understood that the time constants of each of the smoothing functions 20, 22, 24 increase such that smoothing function 3, element 24, has a longer time constant than does smoothing function 1, element 20.

The smoothed signals S1...S3 can in turn be processed further by circuitry 18 so as to generate an output signal on a line 28 usable to determine the presence of a preselected condition. The processed output signal on the line 28 can in turn be compared to a threshold value in a comparator 32 to establish the presence of the predetermined condition, such as products of combustion, pollution or the like.

The output from the comparator 30 on the line 32 is an indication of the presence, or absence of the preselected ambient condition. It will be understood that the comparator 30 could be implemented either in hard-wired circuitry or by prestored instructions in a programmable processor.

Figure 2 illustrates various signals associated with the system 10. The sensor output signal R is illustrated dropping over a period of time in response to a sensed ambient airborne characteristic. Smoothed signals S1, S2 and S3 are illustrated each having an increasing time constant.

As illustrated in Fig. 2, as the output R from the sensor begins to drop, a disparity between the slopes of signal S2 and S2 becomes apparent at "A" seconds. The disparity in slopes can be used to establish the presence of a predetermined ambient condition as discussed subsequently.

As the output from the sensor 12 continues to drop, a disparity also develops between the amplitudes of the processed signals S1 and S3 as illustrated at time "B" and subsequently at time "C".

As a result of the activity of processing circuitry 18, the disparity and amplitudes of the smoothed signals S1 and S3 at "B" contributes to extending the duration of the event indicating output on the line 28. Subsequently, at "C", while the slopes of smoothed signals S1 and S2 are very similar, there continues to be a difference in amplitude values between smoothed signals S1 and S3 sufficient to again produce an output on the line 28 indicative of the potential presence of the preselected condition.

The smoothing process of elements 20-24 can be carried out in hardware using hard-wired RC exponential-type filters for example. It can also be implemented digitally by means of exponential-type smoothing equations.

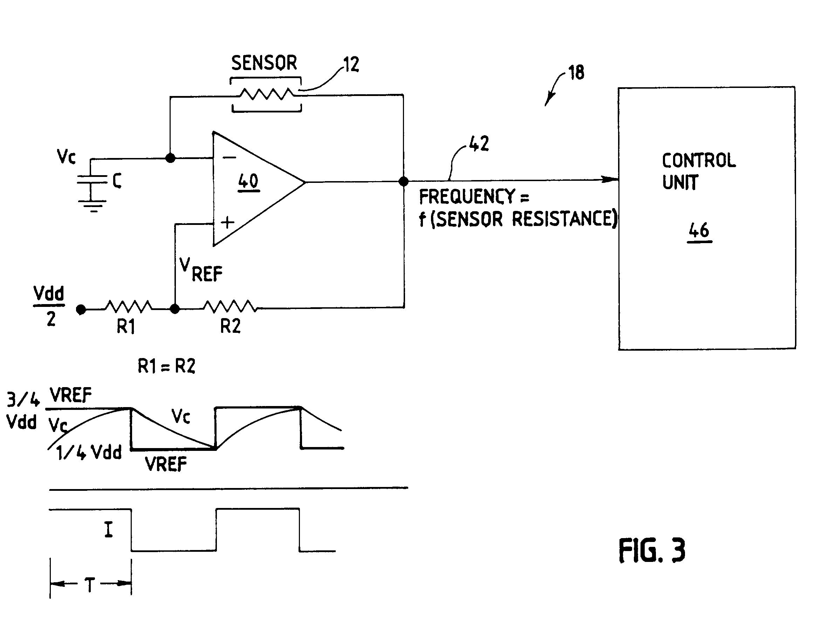

Figure 3 is a schematic block diagram of the system 10 illustrating details of one embodiment of processing circuitry 18. In the embodiment illustrated in Figure 3, sensor 12 is coupled to a negative feedback path of an amplifier 40. The combination of sensor 12, amplifier 40 coupled to capacitor C and resisters R1 and R2 as illustrated in Fig. 3 produces a wide ranging variable frequency oscillator with an output frequency on a line 42 which is a function of the resistance of the sensor 12 (assuming that the sensor 12 is of the type disclosed in the '922 patent previously noted). That frequency is a function of the time to charge and discharge the capacitor C between $V_{REF}$ thresholds. The variable output frequency on the line 42 is in turn coupled to control unit 46 which could be hard-wired or include a programmable processor, such as a microprocessor programmed to carry out the required smoothing functions.

The control unit 46 samples the output of the oscillator on the line 42 at a predetermined sample rate. The waveform on the line 42 can also be monitored for symmetry which can in turn be an indication of any humidity effects on the sensor 12.

The control unit 46 can in turn carry out the required smoothing functions for each sample received from the sensor as follows:

A = predetermined exponential value
T1 = time that the waveform is high
T2 = time that the waveform is low
R = sensor resistance
S1 = .8*S1 + .2*R
S2 = .999*S2 + .001*S1
S3 = .95*S2 + .05*S1
IF S3 > =S2 THEN OUTPUT = (S1/S3 - 1)*(S1/S2 - 1)*(T1/T2)^A
IF S3 <S2 THEN OUTPUT = (S3/S1 -1)*(S2/S1 - 1)*(T1/T2)^A

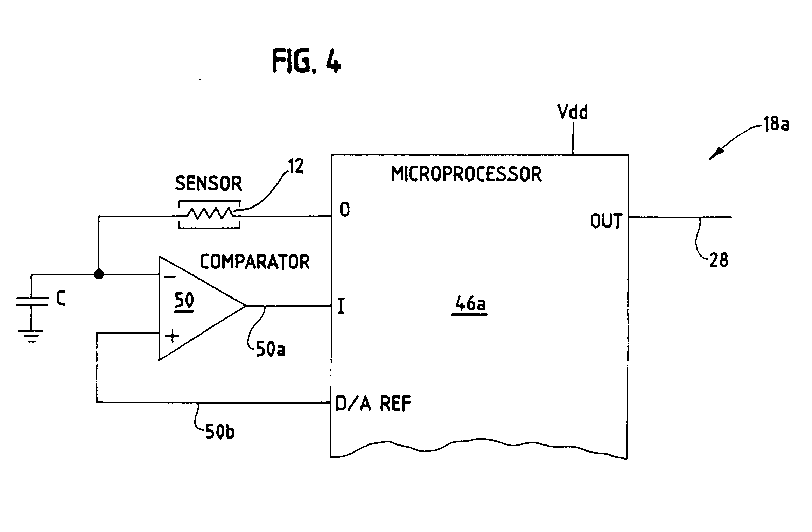

Figure 4 illustrates an alternate form of processing circuitry 18a. In the embodiment of Fig. 4, sensor 12 is coupled between an output of a microprocessor 46a and a negative input of comparator 50. Comparator 50 is in turn coupled to an input of microprocessor 46a on a line 50a as well as an output from the processor 46a on a line 50b.

The circuitry 18a illustrated in Fig. 4 carries out a resistance value to time conversion for purposes of processing the output of sensor 12.

The microprocessor 46a will measure the charge time of capacitor C through the sensor 12. The time to charge the capacitor C is determined by the sensor's resistance. This provides a resistance-to-time conversion for the measurement of the sensor. As the sensor resistance changes with gas concentration, the time will proportionally change with the gas concentration. Thus, a direct measurement of the gas concentration is accomplished as a function of time.

The microprocessor 46a will simultaneously drive the O output and the D/A REF output. The capacitor C is initially discharged to 0 volts. With the O output at Vdd volts, a current starts flowing through the sensor 12 and into the capacitor C to charge it. The D/A REF provides the reference voltage that the capacitor C must charge to for the output of the comparator 50 to switch state and provide the signal to the microprocessor 46a that the charge time is complete. The microprocessor 46a thus measures the time it takes for the capacitor C to charge.

The time it takes to charge is used in equations within the processor to determine an alarm from the gas present.

T = time to charge the capacitor to the D/A REF threshold.
T1 = a*T1 + (1-a)*T which is the first smoothed value.
T2 + b*T2 + (1-b)*T1 which is the second smoothed value.
T3 = c*T3 + (1-c)*T1 which is the third smoothed value.
If T1 > T3 the K = (T1/T2-1)*(T1/T3-1)
    else K = (T2/T1-1)*(T3/T1-1)
If K>1.2 then on line 28, OUT = 1 else OUT = 0

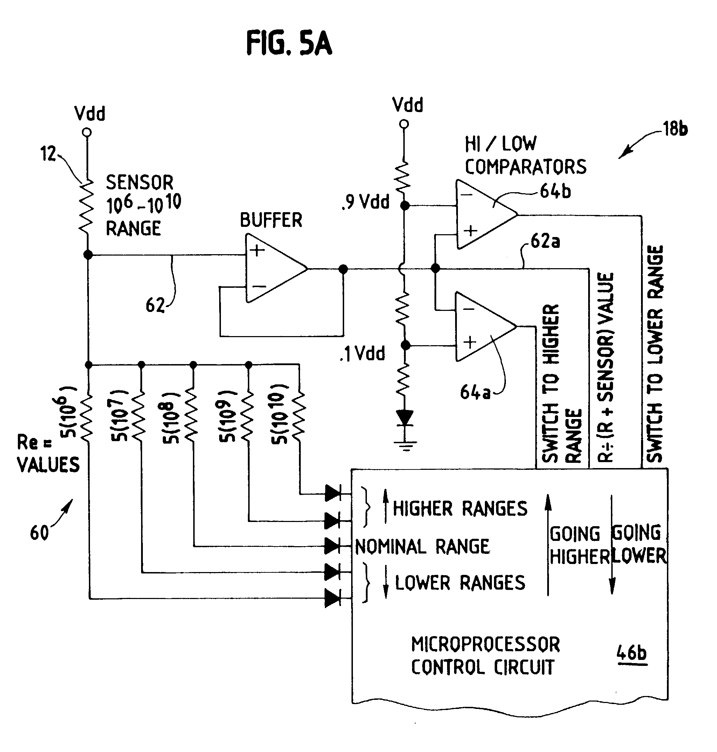

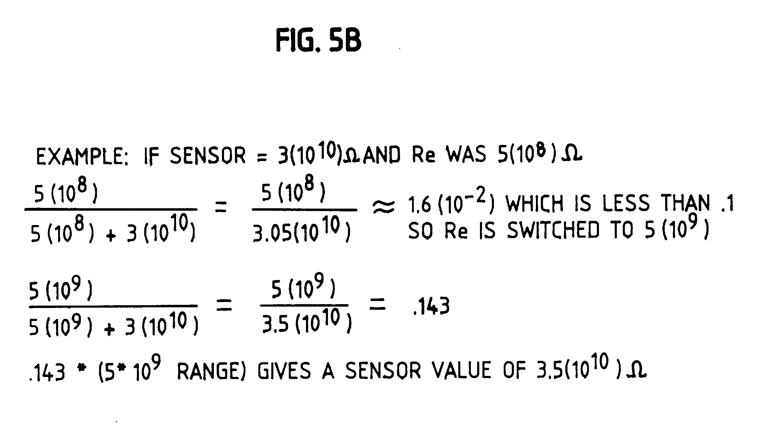

Figures 5A and 5B taken together are a schematic diagram of yet another form of processing circuitry 18b. The embodiment of processing circuitry 18b incorporates an automatically adjustable voltage divider which includes a plurality of resistors 60 coupled to output lines of a programmable processor or hard-wired control circuit 46b. The plurality 60 makes accurate measurements possible over a wide range of sensor resistance values (such as $10^5$-$10^8$ ohms for example). Particular members of the plurality 60 are selected in response to variations in output resistance of the sensor 12.

A gas/fire sensor changes in resistance values very significantly (high/low > 100.) It therefore becomes very important to be able to accurately measure the resistance over a wide dynamic range. However, a simple resistor, divider circuit approach only can handle about a 10:1 change.

The dynamically changing circuit 18b can maintain an accurate resistance measurement over the range output resistance of the sensor 12. The resistance values of the plurality 60, "Re" are automatically switched by the HI/LOW comparators 64a, 6, and control circuit 46b to keep the R/(Re + Sensor) in a range of .1 to .9. Fig. 5B is an example of this process.

The circuit 18b can also be "gated" so that sampling at a specific time is accomplished. Processor 41b can provide the desired gating.

In accordance with the embodiment of processing circuitry 18b, variable resistor from the sensor 12 can be detected by sensing a voltage present at a line 62 which is in turn proportional to the resistance value of the sensor 12. The processor 46b can in turn sample the input voltage on the line 62a at a predetermined rate and carry out the previously described smoothing functions S1...S3 so as to determine a time varying output value indicative of the presence of a predetermined ambient condition.

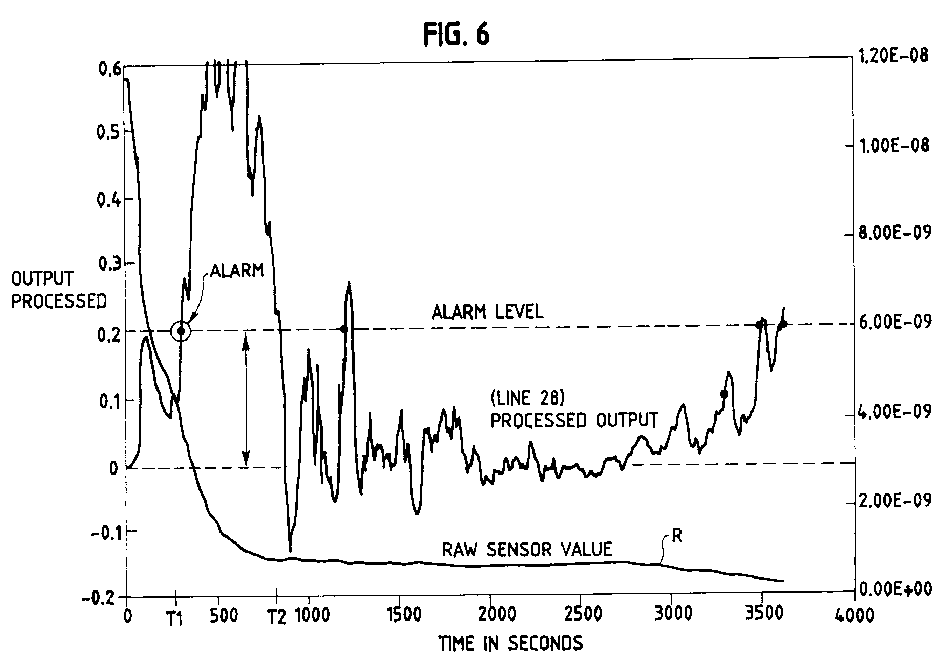

Figure 6 is a graph illustrating output R (such as on line 14) as a function of time as well as processed output (line 28) also as a function of time when processed in accordance with the above-described steps. As illustrated in Fig. 6 as the sensor output value R drops the processed output value:

$$((S2/S1)-1)\times((S3/S1)-1)$$

rises in response to the downward slope of the output signal R as well as the relative change in magnitude thereof and crosses an alarm level threshold, established at the comparator 30 at time T1. An ongoing alarm condition is indicated until time T2.

Subsequently, the output value from the sensor R continues to fall at a relatively low rate in the vicinity of time = 3000 seconds. Nevertheless, as a result of the above-described processing, the processed output signal on the line 28 increases primarily due to relative magnitude changes such that at time corresponding to 3500 seconds the processed output on the line 28 again crosses the alarm threshold level once again indicating an alarm condition during a time when the sensor output value R is dropping at a relatively low rate.

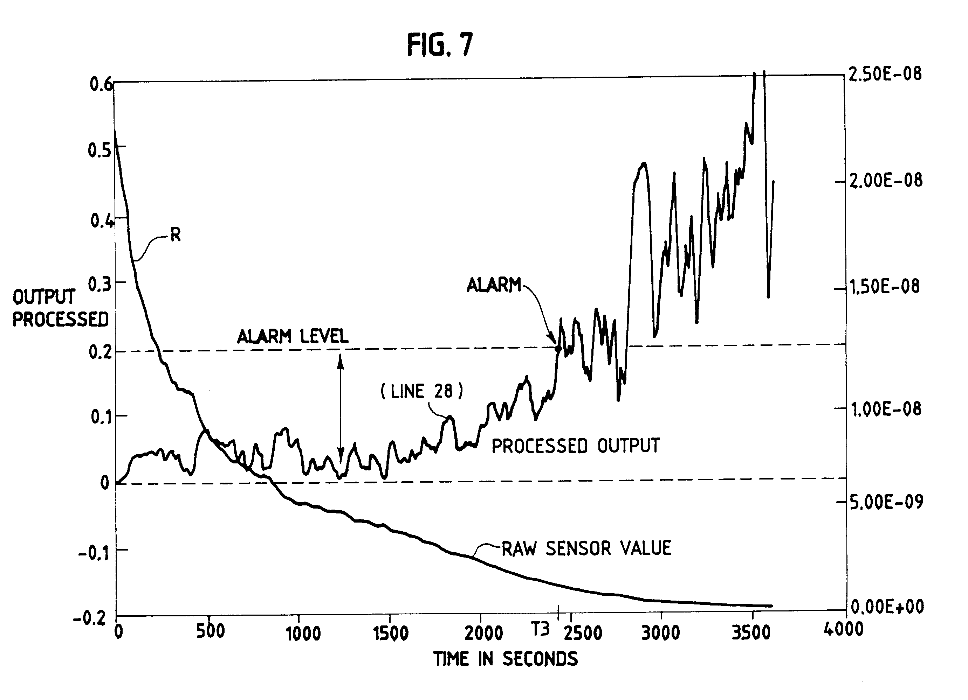

Figure 7 illustrates another graph wherein the sensor output data R might be indicative of a very slow smoldering fire. As is illustrated in Figure 7, at time T3 the process output signal, on line 28, again crossed the alarm threshold indicating an alarm notwithstanding a declining rate of change in resistance value for the sensor 12.

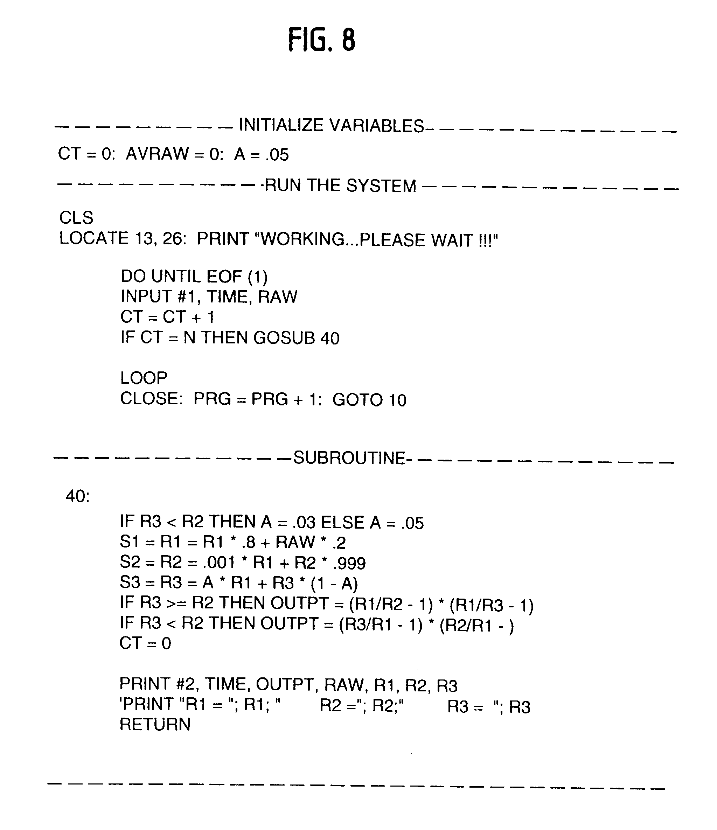

Figure 8 is a listing of a subroutine illustrating steps and a method of processing output signals from sensor 12.

While many of the above comments have been directed toward an ambient condition sensor having a variable resistance characteristic, it will be understood that the invention is not limited to such sensors. Other sensors, without limitation, may be used in combination with processing circuitry of the type described herein.

Additionally, a second sensor, such as a humidity sensor, can be located near the sensor 12 for purposes of taking into account humidity effects. Such effects can be taken into account by carrying out multiple smoothing operations on output values from a respective humidity sensor to form smoothed humidity related output values H1, H2 and H3 similarly to the way the smoothed output values S1, S2 and S3 were formed as described above.

Subsequently, an output value can be generated which reflects minimal humidity effects by forming ratios between respective smoothed values from the sensor 12 and respective smooth humidity values. To take into account differences between the sensors a gain constant K can be incorporated into the processing so as to produce a compensated output value on line 28 as follows:

$$\frac{(S2/H2*K)}{(S1*H1*K)} * \frac{(S3/H3*K)}{(S1/H1*K)} -1$$

From the foregoing, it will be observed that numerous variations and modifications may be effected without departing from the spirit and scope of the invention. It is to be understood that no limitation with respect to the specific apparatus illustrated herein is intended or should be inferred. It is, of course, intended to cover by the appended claims all such modifications as fall within the scope of the claims.

## Claims

1. An apparatus for sensing an ambient condition comprising:

   an ambient condition sensor with a parameter which varies between first and second values in response to the sensed ambient condition; and
   a compensation circuit with a dynamically variable parameter which is adjusted to maintain resolution in measurement of the sensor's parameter as it varies between first and second values.

2. An apparatus as in claim 1 wherein the compensation circuit includes processing circuitry for varying the parameter.

3. An apparatus as in claim 2 wherein the compensation circuit includes at least one solid state element for affecting a flow of current through the sensor.

4. An apparatus as in claim 2 wherein the compensation circuit includes a variable resistance circuit coupled to the processing circuitry.

5. An apparatus as in claim 4 wherein the variable resistance circuit includes at least one variable impedance solid state element.

6. An apparatus as in claim 4 wherein the variable resistance circuit includes a plurality of resistive elements.

7. An apparatus as in claim 1 wherein the parameter corresponds to a resistance value of the sensor and wherein the value of the sensor resistance varies over two or more orders of magnitude in response to the sensed ambient condition.

8. An apparatus as in claim 2 wherein the processing circuitry includes a programmed processor.

9. An apparatus as in claim 1 wherein the relationship between the characteristic and the parameter value is maintained in a predetermined range.

10. An apparatus as in claim 6 wherein the variable resistance circuit includes nonlinear circuitry for coupling the plurality of resistors to the processing circuitry.

11. An apparatus as in claim 6 which includes at least one comparator coupled to the sensor and to the processing circuitry.

12. An apparatus as in claim 1 which includes at least one stage of smoothing circuitry whereby an output signal from the sensor is processed.

13. A method of using an apparatus as in claim 1 which includes the steps of:

sensing an ambient condition and generating an electrical signal indicative, at least in part, thereof;
detecting the electrical signal;
varying at least one characteristic of the compensation circuit in response to the detected signal so as to maintain a parameter of the sensor within a predetermined range.

14. A method as in claim 13 wherein the parameter of the sensor corresponds to an impedance value and the parameter of the compensation circuit corresponds to a second impedance value.

15. A method as in claim 14 wherein a ratio of the second impedance value to the sum of the impedance value plus the second impedance value falls in a predetermined range.

16. A method as in claim 15 wherein the ratio falls within a range of .1 to .9.

17. A method as in claim 14 wherein the generated electrical signal is smoothed.

FIG. 1

FIG. 2

SENSOR 12

Vc

C

40

− +

V_REF

R1

Vdd/2

R2

R1 = R2

42

FREQUENCY = f (SENSOR RESISTANCE)

18

CONTROL UNIT 46

3/4 VREF
Vdd
Vc
Vc
1/4 Vpd
VREF

I

T

FIG. 3

8

# FIG. 4

EP 0 844 477 A1

# FIG. 5A

# FIG. 5B

EXAMPLE:  IF SENSOR $= 3(10^{10})\,\Omega$ AND Re WAS $5(10^8)\,\Omega$

$$\frac{5(10^8)}{5(10^8) + 3(10^{10})} = \frac{5(10^8)}{3.05(10^{10})} \approx 1.6(10^{-2}) \text{ WHICH IS LESS THAN } .1$$

SO Re IS SWITCHED TO $5(10^9)$

$$\frac{5(10^9)}{5(10^9) + 3(10^{10})} = \frac{5(10^9)}{3.5(10^{10})} = .143$$

$.143 * (5 * 10^9$ RANGE) GIVES A SENSOR VALUE OF $3.5(10^{10})\,\Omega$

EP 0 844 477 A1

FIG. 6

## FIG. 7

EP 0 844 477 A1

# FIG. 8

— — — — — — — — — INITIALIZE VARIABLES— — — — — — — — — — — — —

CT = 0:  AVRAW = 0:  A = .05

— — — — — — — — — ·RUN THE SYSTEM — — — — — — — — — — — —

CLS
LOCATE 13, 26:  PRINT "WORKING...PLEASE WAIT !!!"

DO UNTIL EOF (1)
INPUT #1, TIME, RAW
CT = CT + 1
IF CT = N THEN GOSUB 40

LOOP
CLOSE:  PRG = PRG + 1:  GOTO 10

— — — — — — — — — — —SUBROUTINE— — — — — — — — — — — — —

40:

IF R3 < R2 THEN A = .03 ELSE A = .05
S1 = R1 = R1 * .8 + RAW * .2
S2 = R2 = .001 * R1 + R2 * .999
S3 = R3 = A * R1 + R3 * (1 - A)
IF R3 >= R2 THEN OUTPT = (R1/R2 - 1) * (R1/R3 - 1)
IF R3 < R2 THEN OUTPT = (R3/R1 - 1) * (R2/R1 - )
CT = 0

PRINT #2, TIME, OUTPT, RAW, R1, R2, R3
'PRINT "R1 = "; R1; "      R2 ="; R2;"      R3 = "; R3
RETURN

— — — — — — — — — — — — — — — — — — — — — — — — — — —

**European Patent Office**

## EUROPEAN SEARCH REPORT

Application Number

EP 97 30 9379

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | US 4 120 269 A (FUJISHIRO)<br><br>* abstract *<br>* column 3, line 14 - column 5, line 2; figure 2 *<br>--- | 1-5, 13-15 | G01N27/12 |
| A | US 5 531 097 A (TSUCHIDA ET AL.)<br>* abstract *<br>* column 8, line 9 - line 26; figure 1 *<br>--- | 1,13 | |
| A | US 4 306 444 A (HATTORI ET AL.)<br>* abstract *<br>* column 3, line 16 - line 29; figure 6 *<br>----- | 1,13 | |

**TECHNICAL FIELDS SEARCHED** (Int.Cl.6)

G01N

The present search report has been drawn up for all claims

| Place of search | Date of completion of the search | Examiner |
|---|---|---|
| THE HAGUE | 3 February 1998 | Kempf, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)